# EUROPEAN PATENT SPECIFICATION

(11) **EP 4 032 477 B1**
(45) Date of publication and mention of the grant of the patent: **02.10.2024**
(21) Application number: 21182640.9
(22) Date of filing: 30.06.2021
(51) Int. Cl.: A61B 6/03, A61B 6/00

(54) **MEDICAL IMAGING SYSTEM WITH INTEGRATED IN-VITRO-DIAGNOSTIC DEVICE**
MEDIZINISCHES ABBILDUNGSSYSTEM MIT INTEGRIERTER IN-VITRO-DIAGNOSTISCHER VORRICHTUNG
SYSTÈME D'IMAGERIE MÉDICALE AVEC DISPOSITIF DE DIAGNOSTIC IN VITRO INTÉGRÉ

(43) Date of publication of application: 27.07.2022
(73) Proprietor: Siemens Healthineers AG, 91301 Forchheim (DE)
(72) Inventor: Battle, Xavier, 91301 Forchheim (DE); Schmidt, Sebastian, 91085 Weisendorf (DE); Thierfelder, Carsten, 91361 Pinzberg (DE); Zhao, Yufan, 91052 Erlangen (DE)
(74) Representative: Siemens Healthineers Patent Attorneys

(56) References cited:
- US-A1- 2009 069 660
- US-A1- 2010 332 254
- US-A1- 2015 169 001
- US-A1- 2018 268 109

## Description

The invention relates in one aspect to a medical imaging system and in another aspect to a method for operating a medical imaging system.

Patient safety is of the highest importance for our customers. Intravenous iodinated contrast media are commonly used with computed tomography (CT) to evaluate a disease and to determine the treatment response. However Intravenous iodinebased contrast agents used in CT scans can cause acute kidney injury (AKI), particularly in people who are at high risk and those with known kidney dysfunction.

The estimated glomerular filtration rate (eGFR) can be used as an indicator whether the kidneys are working properly. If a person has a low eGFR, intravenous hydration can be offered before the scan to reduce the risk of acute kidney injury. Currently, radiology departments rely on central labs to deliver the eGFR. This could be done, for example, in a hospital which has a central lab, or the patient can do the test through general doctor and bring the result to the CT examination.

If a person does not have a recent eGFR measurement, their CT scan could be cancelled and rescheduled while a creatinine test is processed in the laboratory. For radiology practices without an easily accessible central lab, this may cause wasted CT appointments, delayed diagnosis and, consequently, an increased patient death rate.

However, standalone point-of-care (POC) devices are available, that are configured for a rapid measurement of creatinine levels and a calculation of the estimated glomerular filtration rate (eGFR).

The document US 2009/069660 A1 discloses a medical imaging device comprising a main unit, a data processing unit and an analytical chip connected to the data processing unit.

The underlying technical problem of the invention is to facilitate an improved provision of in-vitro-diagnostic parameters in a medical imaging workflow. This problem is solved by the subject matter of the independent claims. The dependent claims are related to further aspects of the invention.

The invention relates in one aspect to a medical imaging system, comprising
- a medical imaging device for medical imaging of a patient,
- an in-vitro-diagnostic device for in-vitro-diagnostic testing of a sample,
- a control device, wherein the control device is configured to operate the medical imaging device, wherein the control device is further configured to operate the in-vitro-diagnostic device.

The medical imaging of the patient may be a contrast-enhanced medical imaging of the patient. The sample may be a sample of the patient, in particular a biological sample of the patient. The sample may be, for example, a fluid sample, in particular a blood sample or a urine sample. The sample may be, for example, a fingerstick capillary sample. The sample volume may be, for example, less than 250 microliters, in particular, less than 10 microliters.

The medical imaging device may be, for example, a computed tomography (CT) device or a magnetic resonance imaging (MRI) device or a combination of different medical imaging modalities, for example, a PET-CT-imaging device.

In another aspect, the control device is portable and/or comprises a tablet computer. The control device may further comprise a jacket for the tablet computer. Furthermore it can be provided that the tablet computer is embedded in the jacket. In another aspect, the control device and the in-vitro-diagnostic device are attached together to form a portable unit.

In another aspect, the control device comprises a screen, in particular a touch-sensitive screen. A graphical user interface for operating the medical imaging device and/or for operating the in-vitro-diagnostic device and/or for operating the contrast agent injector may be displayed on the screen.

In another aspect, the medical imaging system further comprises a docking structure for accommodating the control device in a releasable form-fitting manner, wherein the docking structure comprises the in-vitro-diagnostic device.

In another aspect, the docking structure comprises a control device interface and a sample holder interface, wherein the control device interface is configured in such a way that the control device is releasably and form-fittingly dockable to the control device interface, wherein the sample holder interface is configured in such a way that a sample holder is releasably and form-fittingly dockable to the sample holder interface, wherein the in-vitro-diagnostic device is configured to perform the in-vitro-diagnostic testing of the sample while the sample is inside the sample holder and the sample holder is releasably and form-fittingly docked to the sample holder interface.

Furthermore it can be provided that the control device interface and the sample holder interface are arranged relative to each other in such a way that a user could simultaneously look at a screen of the tablet computer and manually handle the sample holder, while the control device is releasably and form-fittingly docked to the control device interface and the sample holder is releasably and form-fittingly docked to the sample holder interface.

Furthermore it can be provided that while the sample holder is releasably and form-fittingly docked to the sample holder interface the sample holder is accessible in such a way that the sample can be received by the sample holder.

In another aspect, the sample holder interface and the sample holder are configured in such a way that an electrically conductive connection for electrochemical sensing, for example, amperometric sensing, of the sample is formed between the sample holder interface and the sample holder, upon the sample holder being releasably and form-fittingly docked to the sample holder interface.

In another aspect, the sample holder interface and the sample holder are configured in such a way that an optical connection for optical sensing of the sample is formed between the sample holder interface and the sample holder, upon the sample holder being releasably and form-fittingly docked to the sample holder interface.

Alternatively or additionally to electrochemical sensing and/or optical sensing of the sample, other kinds of sensing known in the art could be used.

In another aspect, the control device interface and the control device are configured in such a way that an electrical power transfer connection for electrical charging of the control device is formed between the control device interface and the control device, upon the control device being releasably and form-fittingly docked to the control device interface. The electrical power transfer connection may be contacted or contactless.

In another aspect, the control device interface and the control device are configured in such a way that a data transfer connection is formed between the control device interface and the control device, upon the control device being releasably and form-fittingly docked to the control device interface. The data transfer connection may be contacted or contactless. The data transfer connection may be, in particular, bidirectional.

In another aspect, the sample holder is card-shaped or stripshaped or tube-shaped. In another aspect, the sample holder is a test cartridge, a test card, a test stripe, or a test tube. The sample holder may comprise, for example, a hollow portion with an opening for receiving the sample, for example, from a syringe.

The sample holder may comprise, for example, a reservoir containing a calibration fluid for calibrating the in-vitro-diagnostic device and/or a reservoir containing an analyte for the in-vitro-diagnostic testing of the sample.

The in-vitro-diagnostic device may comprise an electromechanical actuation assembly for engaging the sample holder after the sample holder is releasably and form-fittingly docked to the sample holder interface. The electro-mechanical actuation assembly may be configured for driving at least one push pin, thereby opening at least one valve in the sample holder, and/or for driving at least one plunger, thereby causing at least one fluid, for example a calibration fluid and/or the fluid sample, to flow across a sensor module within the sample holder. The in-vitro-diagnostic device may include circuits for amplifying, digitizing, and converting the raw sensor signals to a format that can be received and processed by the control device.

The in-vitro-diagnostic device may further comprise a thermal system that provides a temperature-controlled environment for a sensor module of the sample holder during the in-vitro-diagnostic testing of the sample. The thermal system may comprise at least one heater that is mounted for releasably thermal contacting the sample holder, when the sample holder is releasably and form-fittingly docked to the sample holder interface.

The in-vitro-diagnostic device may further comprise a cleaning system for cleaning an exposed part of the in-vitro-diagnostic device. The cleaning system may comprise a reservoir containing a cleaning fluid for cleaning the in-vitro-diagnostic device and/or a plunger for causing the cleaning fluid to flow through the exposed part of the in-vitro-diagnostic device. The exposed part of the in-vitro diagnostic device can be, for example, an interface duct for receiving the sample.

In another aspect, the medical imaging system further comprises a docking station, wherein the docking station is arranged separately from the medical imaging device, wherein the docking station comprises the docking structure. The docking station may be, for example, portable and/or wall mounted.

Furthermore it can be provided that the control device and the docking station together form a portable unit while the control device is releasably and form-fittingly docked to the control device interface.

The medical imaging system may further comprise a scanner for scanning a code, for example, a barcode or a QR code, and/or for scanning a tag, for example, an RFID tag. The sample holder may comprise the code and/or the tag, that can be used, for example, for an identification of the patient. The scanner may be integrated, for example, into the control device, the in-vitro-diagnostic device, the medical imaging device, or the docking station.

In another aspect, the medical imaging device comprises a gantry, for example, a computed tomography gantry, wherein the gantry comprises the docking structure.

In another aspect, the medical imaging device comprises a patient bed, wherein the patient bed comprises the docking structure.

In another aspect, the medical imaging system further comprises a contrast agent injector for injecting a contrast agent into the patient, wherein the medical imaging device comprises a patient support area in which the patient is placeable for the medical imaging of the patient, wherein the medical imaging device further comprises a holding structure for holding the docking structure movably relative to the patient support area, wherein the docking structure further comprises an injector interface, wherein the contrast agent injector is releasably and form-fittingly docked to the injector interface. In another aspect, the control device is further configured to operate the contrast agent injector.

In another aspect, the control device is configured to operate the in-vitro-diagnostic device to detect an in-vitro-diagnostic parameter of the sample through the in-vitro diagnostic testing, wherein the control device is further configured to adapt an operation of the medical imaging device, in particular with regard to examination parameters of the medical imaging, based on the in-vitro-diagnostic parameter of the sample, in particular, without a manual user input of the in-vitro-diagnostic parameter of the sample into a user interface for operating the medical imaging device.

In another aspect, the control device is further configured to adapt an operation of the contrast agent injector based on the in-vitro-diagnostic parameter of the sample, in particular, without a manual user input of the in-vitro-diagnostic parameter of the sample into a user interface for operating the contrast agent injector.

In another aspect, the sample is a blood sample, wherein the control device is configured to operate the in-vitro-diagnostic device to detect a creatinine level of the sample through the in-vitro diagnostic testing. The creatinine level of the sample can be detected, for example, based on amperometry using enzyme electrodes.

The invention relates in another aspect to a method for operating a medical imaging system, the medical imaging system comprising a medical imaging device for medical imaging of a patient, an in-vitro-diagnostic device for in-vitro-diagnostic testing of a sample and a control device, the method comprising:
- an operating the in-vitro-diagnostic device by the control device and
- an operating the medical imaging device by the control device.

In another aspect, the medical imaging system further comprises a docking structure, wherein the docking structure comprises the in-vitro-diagnostic device, a control device interface, and a sample holder interface, wherein the method further comprises:
- a docking the control device releasably and form-fittingly to the control device interface,
- a docking a sample holder releasably and form-fittingly to the sample holder interface,
- an in-vitro-diagnostic testing of the sample by the in-vitro-diagnostic device while the sample is inside the sample holder and the sample holder is releasably and form-fittingly docked to the sample holder interface.

Furthermore it can be provided that the method comprises a receiving the sample by the sample holder while the sample holder is releasably and form-fittingly docked to the sample holder interface.

Furthermore it can be provided that the method comprises a displaying an information regarding the in-vitro-diagnostic testing on a screen of the control device while the control device is releasably and form-fittingly docked to the control device interface and the sample holder is releasably and form-fittingly docked to the sample holder interface.

In another aspect, the operating the in-vitro-diagnostic device by the control device comprises a detecting an in-vitro-diagnostic parameter of the sample through the in-vitro diagnostic testing.

In another aspect, the method further comprises an adapting, by the control device, an operation of the medical imaging device based on the in-vitro-diagnostic parameter of the sample.

In another aspect, the sample is a blood sample, wherein the in-vitro-diagnostic parameter of the sample is a creatinine level of the sample, wherein the method further comprises
- a calculating, by the control device, an estimated glomerular filtration rate based on the creatinine level of the sample,
- a providing, by the control device, the estimated glomerular filtration rate.

Furthermore it can be provided that the method comprises a receiving, by the control device, patient data, wherein the patient data are related to the patient. The adapting, by the control device, the operation of the medical imaging device may be further based on the patient data. The calculating, by the control device, the estimated glomerular filtration rate may be further based on the patient data.

The in-vitro-diagnostic parameter of the sample and/or the estimated glomerular filtration rate may be provided, by the control device, for example, by being displayed on a screen of the control device.

The in-vitro-diagnostic device can be used, for example, in a radiology preparation room, a control room and/or a scan room. With the proposed solution, patient safety, patient experience and user experience can be improved, in particular with regard to automated workflow and precision medicine.

Data, in particular the patient data, can be received, for example, by receiving a signal that carries the data and/or by reading the data from a computer-readable medium and/or by receiving an input through a user interface. Data, in particular, the estimated glomerular filtration rate, can be provided, for example, by transmitting a signal that carries the data and/or by writing the data into a computer-readable medium and/or by displaying the data on a display.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Reference is made to the fact that the described methods and the described units are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a docking station comprising the in-vitro-diagnostic device, wherein a control device is releasably and form-fittingly docked to a control device interface of the docking station.
Fig. 2 shows the docking station with the control device removed.
Fig. 3 shows another example of a docking station comprising the in-vitro diagnostic device.
Fig. 4 shows a medical imaging system comprising a medical imaging device, an in-vitro-diagnostic device, and a control device.
Fig. 5 shows a portion of a gantry of a medical imaging device, wherein the gantry comprises the in-vitro-diagnostic device.
Fig. 6 shows a diagram illustrating a method for operating a medical imaging system.
Fig. 7 shows a diagram illustrating another example of a method for operating a medical imaging system.

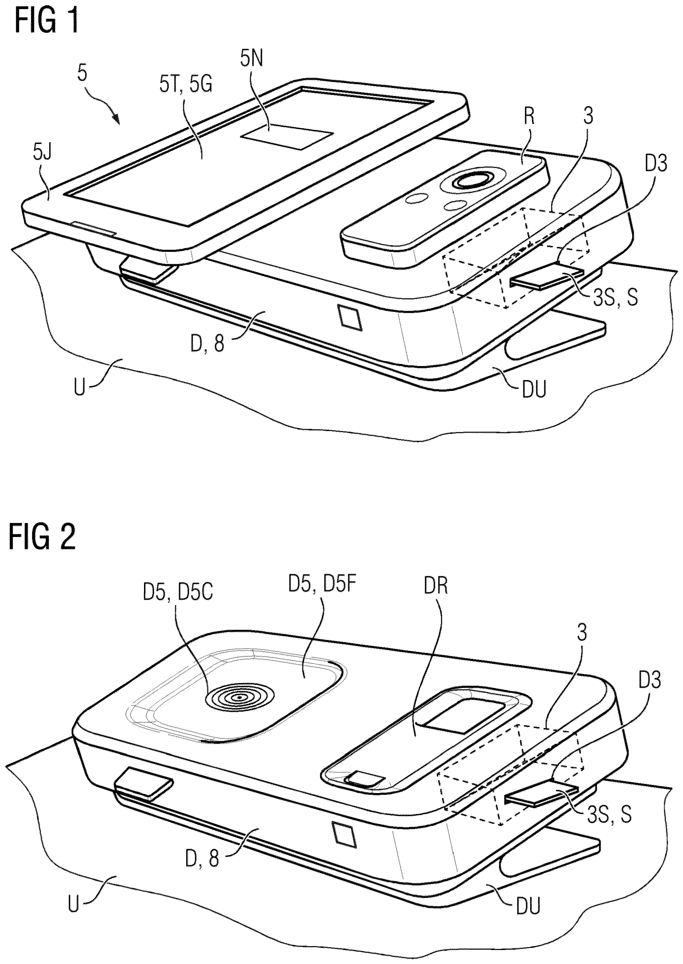

Fig. 1 shows a docking station 8 comprising the in-vitro-diagnostic device 3 for in-vitro-diagnostic testing of a sample S, wherein the control device 5 is releasably and form-fittingly docked to the control device interface D5 of the docking station 8. The additional remote control device R comprising at least one electromechanical switch for triggering and/or interrupting radiation exposure by the medical imaging device 2 is releasably and form-fittingly docked to the remote-control interface DR of the docking station 8.

The control device 5 is configured to operate the medical imaging device 2, wherein the control device 5 is further configured to operate the in-vitro-diagnostic device 3. The control device 5 is portable and comprises a tablet computer 5T. The control device 5 further comprises a jacket 5J for the tablet computer 5T. The tablet computer 5T is embedded in the jacket 5J.

The docking station 8 shown in Fig. 1 is arranged separately from the medical imaging device 2 and comprises the docking structure D for accommodating the control device 5 in a releasable form-fitting manner, wherein the docking structure D comprises the in-vitro-diagnostic device 3. The docking station 8 is arranged on the desk U using the portable support structure DU. The desk U may be, for example, a control room scan desk. The docking station 8 is connected to an electrical power grid through the cable DL.

Fig. 2 shows the docking station 8 with the control device 5 and the additional remote control device R removed.

The docking structure D comprises a control device interface D5 and a sample holder interface D3, wherein the control device interface D5 is configured in such a way that the control device 5 is releasably and form-fittingly dockable to the control device interface D5, wherein the sample holder interface D3 is configured in such a way that a sample holder 3S is releasably and form-fittingly dockable to the sample holder interface D3, wherein the in-vitro-diagnostic device 3 is configured to perform the in-vitro-diagnostic testing of the sample S while the sample S is inside the sample holder 3S and the sample holder 3S is releasably and form-fittingly docked to the sample holder interface D3.

The control device interface D5 and the sample holder interface D3 are arranged relative to each other in such a way that a user U1 could simultaneously look at the screen 5G of the tablet computer 5T and manually handle the sample holder 3S, while the control device 5 is releasably and form-fittingly docked to the control device interface D5 and the sample holder 3S is releasably and form-fittingly docked to the sample holder interface D3. While the sample holder 3S is releasably and form-fittingly docked to the sample holder interface D3 the sample holder 3S is accessible in such a way that the sample S can be received by the sample holder 3S. The sample holder 3S is card-shaped.

The control device interface D5 comprises a recess D5F for releasably and form-fittingly accommodating the control device 5. The control device interface D5 further comprises an electrical power transfer interface for connection to a corresponding counterpart of the control device 5 to form an electrical power transfer connection for electrical charging of the control device 5, upon the control device 5 being releasably and form-fittingly docked to the control device interface D5.

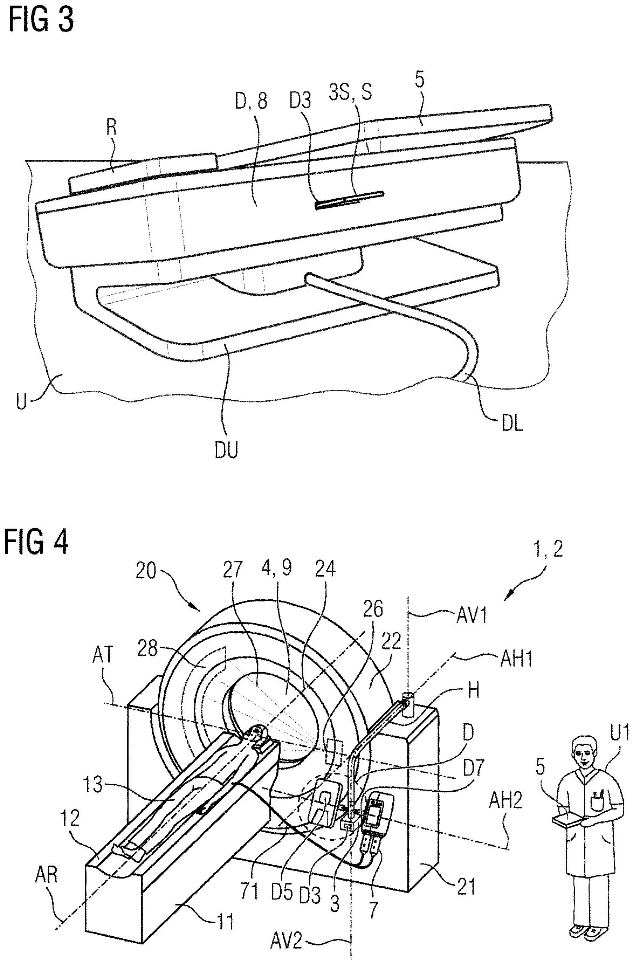

Fig. 3 shows another example of a docking station 8 comprising the in-vitro diagnostic device 3.

Fig. 4 shows a medical imaging system 1 comprising the medical imaging device 2 for medical imaging of the patient 13, the in-vitro-diagnostic device 3 and the control device 5.

The medical imaging device 2 shown in Fig. 4 is a computed tomography device and comprises the gantry 20 and the patient bed 11. The patient 13 is placed in the patient support area 12 on the patient bed 11, the patient bed 11 being longitudinally extendable so that the patient 13 can be inserted into the examination area 4 within the opening 9 of the gantry 20. The gantry 20 comprises the support frame 21 and the tilting frame 22 that is mounted tiltable relative to the support frame 21 around the tilt axis AT. The gantry 20 further comprises the rotating frame 24 that is mounted rotatable relative to the tilting frame 22 around the rotation axis AR. A radiation source 24 for producing radiation 27 and a radiation detector 28 for detecting the radiation 27 are mounted on the rotating frame 24.

The medical imaging system 1 comprises a contrast agent injector 7 for injecting a contrast agent into the patient 13, wherein the medical imaging device 2 comprises a patient support area 12 in which the patient 13 is placeable for the medical imaging of the patient 13, wherein the medical imaging device 2 further comprises a holding structure H for holding the docking structure D movably relative to the patient support area 12, wherein the docking structure D further comprises an injector interface D7, wherein the contrast agent injector 7 is releasably and form-fittingly docked to the injector interface D7.

The holding structure H is arm-shaped and mounted rotatable relative to the gantry 20 around the first vertical axis AV1 and tiltable relative to the gantry 20 around the first horizontal axis AH1. The docking structure D is mounted rotatable relative to the holding structure H around the second vertical axis AV2. When docked to the docking structure D, the control device 5 and the contrast agent injector 7 are tiltable relative to the holding structure H around the second horizontal axis AH2 independently from each other. The contrast agent injector 7 is connected to the patient 13 through the tubes 71.

The control device 5 is configured to operate the in-vitro-diagnostic device 3 to detect an in-vitro-diagnostic parameter of the sample S through the in-vitro diagnostic testing, wherein the control device 5 is further configured to adapt an operation of the medical imaging device 2 based on the in-vitro-diagnostic parameter of the sample S. The sample S is a blood sample and the control device 5 is configured to operate the in-vitro-diagnostic device 3 to detect a creatinine level of the sample S through the in-vitro diagnostic testing.

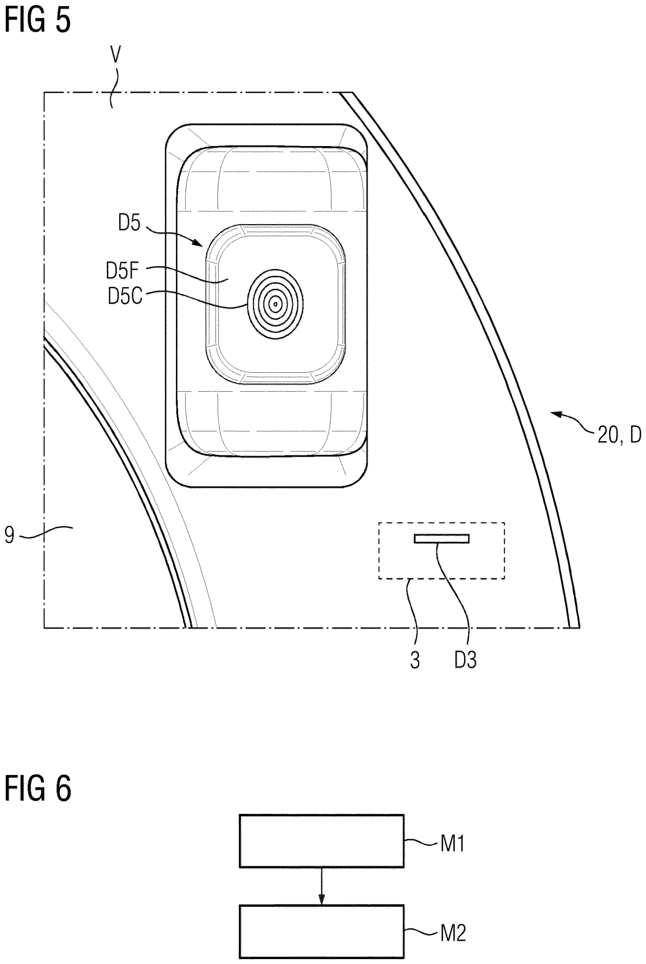

Fig. 5 shows a portion of a gantry 20 of a medical imaging device 2, wherein the gantry 20 comprises the in-vitro-diagnostic device 3 and the docking structure D. The control device interface D5 and the sample holder interface D3 are integrated into the cover V of the gantry 20.

Fig. 6 shows a diagram illustrating a method for operating a medical imaging system 1, the medical imaging system 1 comprising a medical imaging device 2 for medical imaging of a patient 13, an in-vitro-diagnostic device 3 for in-vitro-diagnostic testing of a sample S and a control device 5, the method comprising:
- an operating M1 the in-vitro-diagnostic device 3 by the control device 5 and
- an operating M2 the medical imaging device 2 by the control device 5.

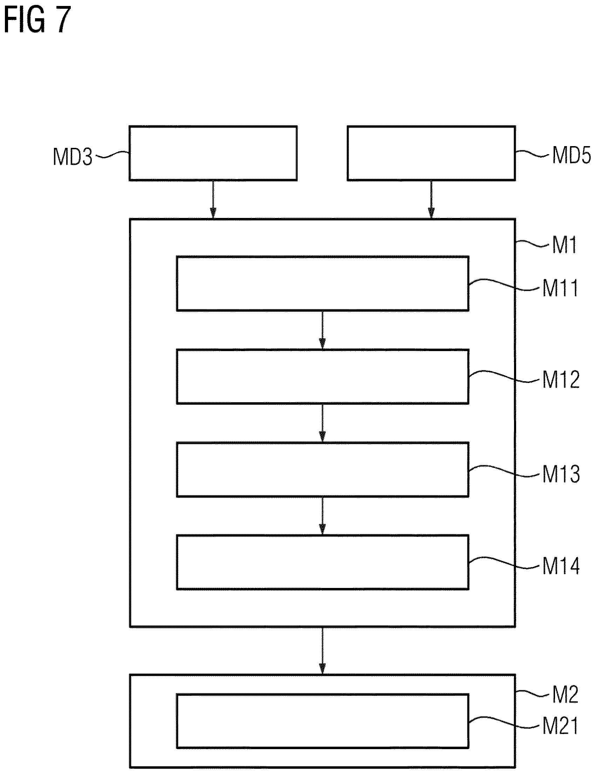

Fig. 7 shows a diagram illustrating another example of a method for operating the medical imaging system 1, wherein the medical imaging system 1 further comprises a docking structure D, wherein the docking structure D comprises the in-vitro-diagnostic device 3, a control device interface D5 and a sample holder interface D3, wherein the method further comprises:
- a docking MD5 the control device 5 releasably and form-fittingly to the control device interface D5,
- a docking MD3 a sample holder 3S releasably and form-fittingly to the sample holder interface D3,
- an in-vitro-diagnostic testing M11 of the sample S by the in-vitro-diagnostic device 3 while the sample S is inside the sample holder 3S and the sample holder 3S is releasably and form-fittingly docked to the sample holder interface D3.

An information 5N regarding the in-vitro-diagnostic testing is displayed on the screen 5G of the control device 5 while the control device 5 is releasably and form-fittingly docked to the control device interface D5 and the sample holder 3S is releasably and form-fittingly docked to the sample holder interface D3.

The operating M1 the in-vitro-diagnostic device 3 by the control device 5 comprises a detecting M12 an in-vitro-diagnostic parameter of the sample S through the in-vitro diagnostic testing M11.

The method further comprises an adapting M21, by the control device 5, an operation of the medical imaging device 2 based on the in-vitro-diagnostic parameter of the sample S. The in-vitro-diagnostic parameter of the sample S is a creatinine level of the sample S, wherein the method further comprises a calculating M13, by the control device 5, an estimated glomerular filtration rate based on the creatinine level of the sample S and a providing M14, by the control device 5, the estimated glomerular filtration rate.

## Claims

1. A medical imaging system (1), comprising
- a medical imaging device (2) for medical imaging of a patient (13),
- an in-vitro-diagnostic device (3) for in-vitro-diagnostic testing of a sample (S),
- a control device (5), wherein the control device (5) is portable and configured to operate the medical imaging device (2), wherein the control device (5) is further configured to operate the in-vitro-diagnostic device (3),
- a docking station (8), wherein the docking station (8) is portable and arranged separately from the medical imaging device (2), wherein the docking station (8) comprises a docking structure (D) for accommodating the control device (5) in a releasable form-fitting manner, wherein the docking structure (D) comprises the in-vitro-diagnostic device (3).

2. The medical imaging system (1) according to claim 1,
- wherein the control device (5) comprises a tablet computer (5T) .

3. The medical imaging system (1) according to claim 1 or 2,
- wherein the docking structure (D) comprises a control device interface (D5) and a sample holder interface (D3),
- wherein the control device interface (D5) is configured in such a way that the control device (5) is releasably and form-fittingly dockable to the control device interface (D5),
- wherein the sample holder interface (D3) is configured in such a way that a sample holder (3S) is releasably and form-fittingly dockable to the sample holder interface (D3),
- wherein the in-vitro-diagnostic device (3) is configured to perform the in-vitro-diagnostic testing of the sample (S) while the sample (S) is inside the sample holder (3S) and the sample holder (3S) is releasably and form-fittingly docked to the sample holder interface (D3).

4. The medical imaging system (1) according to claim 3,
- wherein the sample holder (3S) is card-shaped or stripshaped or tube-shaped.

5. The medical imaging system (1) according to one of the claims 1 to 4,
- wherein the control device (5) is configured to operate the in-vitro-diagnostic device (3) to detect an in-vitro-diagnostic parameter of the sample (S) through the in-vitro diagnostic testing,
- wherein the control device (5) is further configured to adapt an operation of the medical imaging device (2) based on the in-vitro-diagnostic parameter of the sample (S).

6. The medical imaging system (1) according to one of the claims 1 to 5,
- wherein the sample (S) is a blood sample,
- wherein the control device (5) is configured to operate the in-vitro-diagnostic device (3) to detect a creatinine level of the sample (S) through the in-vitro diagnostic testing.

7. A method for operating a medical imaging system (1) according to claim 1 or 2, the method comprising:
- an operating (M1) the in-vitro-diagnostic device (3) by the control device (5) and
- an operating (M2) the medical imaging device (2) by the control device (5).

8. The method according to claim 7, wherein the medical imaging system (1) further comprises a docking structure (D), wherein the docking structure (D) comprises the in-vitro-diagnostic device (3), a control device interface (D5) and a sample holder interface (D3), wherein the method further comprises:
- a docking (MD5) the control device (5) releasably and form-fittingly to the control device interface (D5),
- a docking (MD3) a sample holder (3S) releasably and form-fittingly to the sample holder interface (D3),
- an in-vitro-diagnostic testing (M11) of the sample (S) by the in-vitro-diagnostic device (3) while the sample (S) is inside the sample holder (3S) and the sample holder (3S) is releasably and form-fittingly docked to the sample holder interface (D3).

9. The method according to claim 7 or 8,
- wherein the operating (M1) the in-vitro-diagnostic device (3) by the control device (5) comprises a detecting (M12) an in-vitro-diagnostic parameter of the sample (S) through the in-vitro diagnostic testing (M11).

10. The method according to claim 9, further comprising:
- an adapting (M21), by the control device (5), an operation of the medical imaging device (2) based on the in-vitro-diagnostic parameter of the sample (S).

11. The method according to claim 9 or 10, wherein the sample (S) is a blood sample, wherein the in-vitro-diagnostic parameter of the sample (S) is a creatinine level of the sample (S), wherein the method further comprises
- a calculating (M13), by the control device (5), an estimated glomerular filtration rate based on the creatinine level of the sample (S),
- a providing (M14), by the control device (5), the estimated glomerular filtration rate.

## Patentansprüche

1. Medizinisches Bildgebungssystem (1), umfassend:
- eine medizinische Bildgebungsvorrichtung (2) zur medizinischen Bildgebung eines Patienten (13),
- eine In-Vitro-Diagnosevorrichtung (3) für In-Vitro-Diagnosetests einer Probe (S),
- eine Steuerungsvorrichtung (5), wobei die Steuerungsvorrichtung (5) tragbar ist und dazu ausgelegt ist, die medizinische Bildgebungsvorrichtung (2) zu betreiben, wobei die Steuerungsvorrichtung (5) ferner dazu ausgelegt ist, die In-Vitro-Diagnosevorrichtung (3) zu betreiben,
- eine Docking-Station (8), wobei die Docking-Station (8) tragbar ist und getrennt von der medizinischen Bildgebungsvorrichtung (2) angeordnet ist, wobei die Docking-Station (8) eine Docking-Struktur (D) zum Unterbringen der Steuerungsvorrichtung (5) in lösbarer formschlüssiger Weise aufweist, wobei die Docking-Struktur (D) die In-Vitro-Diagnosevorrichtung (3) umfasst.

2. Medizinisches Bildgebungssystem (1) gemäß Anspruch 1,
- wobei die Steuerungsvorrichtung (5) einen Tablet-Computer (5T) umfasst.

3. Medizinisches Bildgebungssystem (1) gemäß Anspruch 1 oder 2,
- wobei die Docking-Struktur (D) eine Steuerungsvorrichtung-Schnittstelle (D5) und eine Probenhalter-Schnittstelle (D3) umfasst,
- wobei die Steuerungsvorrichtung-Schnittstelle (D5) dergestalt ausgelegt ist, dass die Steuerungsvorrichtung (5) lösbar und formschlüssig an die Steuerungsvorrichtung-Schnittstelle (D5) angedockt werden kann,
- wobei die Probenhalter-Schnittstelle (D3) dergestalt ausgelegt ist, dass ein Probenhalter (3S) lösbar und formschlüssig an die Probenhalter-Schnittstelle (D3) angedockt werden kann,
- wobei die In-Vitro-Diagnosevorrichtung (3) dazu ausgelegt ist, die In-Vitro-Diagnosetests der Probe (S) durchzuführen, während sich die Probe (S) in dem Probenhalter (3S) befindet, und der Probenhalter (3S) lösbar und formschlüssig an die Probenhalter-Schnittstelle (D3) angedockt werden kann.

4. Medizinisches Bildgebungssystem (1) gemäß Anspruch 3,
- wobei der Probenhalter (3S) kartenförmig oder streifenförmig oder röhrenförmig ist.

5. Medizinisches Bildgebungssystem (1) gemäß einem der Ansprüche 1 bis 4,
- wobei die Steuerungsvorrichtung (5) dazu ausgelegt ist, die In-Vitro-Diagnosevorrichtung (3) zu betreiben, um einen In-Vitro-Diagnoseparameter der Probe (S) durch die In-Vitro-Diagnosetests zu detektieren,
- wobei die Steuerungsvorrichtung (5) ferner dazu ausgelegt ist, einen Betrieb der medizinischen Bildgebungsvorrichtung (2) basierend auf dem In-Vitro-Diagnoseparameter der Probe (S) anzupassen.

6. Medizinisches Bildgebungssystem (1) gemäß einem der Ansprüche 1 bis 5,
- wobei die Probe (S) eine Blutprobe ist,
- wobei die Steuerungsvorrichtung (5) dazu ausgelegt ist, die In-Vitro-Diagnosevorrichtung (3) zu betreiben, um ein Kreatininniveau der Probe (S) durch die In-Vitro-Diagnosetests zu detektieren.

7. Verfahren zum Betreiben eines medizinischen Bildgebungssystems (1) gemäß Anspruch 1 oder 2, wobei das Verfahren umfasst:
- Betreiben (M1) der In-Vitro-Diagnosevorrichtung (3) durch die Steuerungsvorrichtung (5) und
- Betreiben (M2) der medizinischen Bildgebungsvorrichtung (2) durch die Steuerungsvorrichtung (5).

8. Verfahren gemäß Anspruch 7, wobei das medizinische Bildgebungssystem (1) ferner eine Docking-Struktur (D) umfasst, wobei die Docking-Station (D) die In-Vitro-Diagnosevorrichtung (3), eine Steuerungsvorrichtung-Schnittstelle (D5) und eine Probenhalter-Schnittstelle (D3) umfasst, wobei das Verfahren ferner umfasst:
- Andocken (MD5) der Steuerungsvorrichtung (5), lösbar und formschlüssig, an die Steuerungsvorrichtung-Schnittstelle (D5),
- Andocken (MD3) eines Probenhalters (3S), lösbar und formschlüssig, an die Probenhalter-Schnittstelle (D3),
- In-Vitro-Diagnosetests (M11) der Probe (S) durch die In-Vitro-Diagnosevorrichtung (3), während sich die Probe (S) in dem Probenhalter (3S) befindet und der Probenhalter (3S) lösbar und formschlüssig an die Probenhalter-Schnittstelle (D3) angedockt ist.

9. Verfahren gemäß Anspruch 7 oder 8,
- wobei das Betreiben (M1) der In-Vitro-Diagnosevorrichtung (3) durch die Steuerungsvorrichtung (5) das Detektieren (M12) eines In-Vitro-Diagnoseparameters der Probe (S) durch die In-Vitro-Diagnosetests (M11) umfasst.

10. Verfahren gemäß Anspruch 9, ferner umfassend:
- Anpassen (M21), durch die Steuerungsvorrichtung (5), eines Betriebs der medizinischen Bildgebungsvorrichtung (2) basierend auf dem In-Vitro-Diagnoseparameter der Probe (S).

11. Verfahren gemäß Anspruch 9 oder 10, wobei die Probe (S) eine Blutprobe ist, wobei der In-Vitro-Diagnoseparameter der Probe (S) ein Kreatininniveau der Probe (S) ist, wobei das Verfahren ferner umfasst:
- Berechnen (M13), durch die Steuerungsvorrichtung (5), einer geschätzten glomerulären Filtrationsrate basierend auf dem Kreatininniveau der Probe (S),
- Bereitstellen (M14), durch die Steuerungsvorrichtung (5), der geschätzten glomerulären Filtrationsrate.

## Revendications

1. Système (1) d'imagerie médicale, comprenant
- un dispositif (2) d'imagerie médicale pour l'imagerie médicale d'un patient (13),
- un dispositif (3) de diagnostic in-vitro pour un test de diagnostic in-vitro d'un échantillon (S),
- un dispositif (5) de commande, dans lequel le dispositif (5) de commande est portatif et est configuré pour faire fonctionner le dispositif (2) d'imagerie médicale, dans lequel le dispositif (5) de commande est configuré en outre pour faire fonctionner le dispositif (3) de diagnostic in-vitro,
- un poste (8) d'accueil, dans lequel le poste (8) d'accueil est portatif et agencé séparément du dispositif (2) d'imagerie médicale, dans lequel le poste (8) d'accueil comprend une structure (D) d'accueil pour loger le dispositif (5) de commande, d'une manière à adaptation de forme libérable, dans lequel la structure (D) d'accueil comprend le dispositif (3) de diagnostic in-vitro.

2. Système (1) d'imagerie médicale suivant la revendication 1,
- dans lequel le dispositif (5) de commande comprend un ordinateur (5T) à tablette.

3. Système (1) d'imagerie médicale suivant la revendication 1 ou 2,
- dans lequel la structure (D) d'accueil comprend une interface (D5) de dispositif de commande et une interface (D3) de support d'échantillon,
- dans lequel l'interface (D5) de dispositif de commande est configurée, de manière à ce que le dispositif (5) de commande puisse être accueilli de manière libérable et à adaptation de forme à l'interface (D5) de dispositif de commande,
- dans lequel l'interface (D3) de support d'échantillon est configurée, de manière à ce qu'un support (3S) d'échantillon puisse être accueilli de manière libérable et à adaptation de forme à l'interface (D3) de support d'échantillon,
- dans lequel le dispositif (3) de diagnostic in-vitro est configuré pour effectuer le test de diagnostic in-vitro de l'échantillon (S), alors que l'échantillon (S) est à l'intérieur du support (3S) d'échantillon et alors que le support (3S) d'échantillon est accueilli de manière libérable et à adaptation de forme à l'interface (D3) de support d'échantillon.

4. Système (1) d'imagerie médicale suivant la revendication 3,
- dans lequel le support (3S) d'échantillon est en forme de carte, en forme de bande ou en forme de tube.

5. Système (1) d'imagerie médicale suivant l'une des revendications 1 à 4,
- dans lequel le dispositif (5) de commande est configuré pour faire fonctionner le dispositif (3) de diagnostic in-vitro, afin de détecter un paramètre de diagnostic in-vitro de l'échantillon (S) par le test de diagnostic in-vitro,
- dans lequel le dispositif (5) de commande est configuré en outre pour adapter un fonctionnement du dispositif (2) d'imagerie médicale sur la base du paramètre de diagnostic in-vitro de l'échantillon (S).

6. Système (1) d'imagerie médicale suivant l'une des revendications 1 à 5,
- dans lequel l'échantillon (S) est un échantillon de sang,
- dans lequel le dispositif (5) de commande est configuré pour faire fonctionner le dispositif (3) de diagnostic in-vitro, afin de détecter un niveau de créatinine de l'échantillon (S) par le test de diagnostic in-vitro.

7. Procédé pour faire fonctionner un système (1) d'imagerie médicale suivant la revendication 1 ou 2, le procédé comprenant :
- une mise en fonctionnement (M1) du dispositif (3) de diagnostic in-vitro par le dispositif (5) de commande et
- une mise en fonctionnement (M2) du dispositif (2) d'imagerie médicale par le dispositif (5) de commande.

8. Procédé suivant la revendication 7, dans lequel le système (1) d'imagerie médicale comprend en outre une structure (D) d'accueil, dans lequel la structure (D) d'accueil comprend le dispositif (3) de diagnostic in-vitro, une interface (D5) de dispositif de commande et une interface (D3) de support d'échantillon, dans lequel le procédé comprend en outre :
- un accueil (MD5) du dispositif (5) de commande, de manière libérable et à adaptation de forme à l'interface (D5) du dispositif de commande,
- un accueil (MD3) d'un support (3S) d'échantillon, de manière libérable et à adaptation de forme à l'interface (D3) de support d'échantillon,
- un test (M11) de diagnostic in-vitro de l'échantillon (S) dans le dispositif (3) de diagnostic in-vitro, alors que l'échantillon (S) est à l'intérieur du support (3S) d'échantillon et alors que le support (3S) d'échantillon est accueilli de manière libérable et à adaptation de forme à l'interface (D3) de support d'échantillon.

9. Procédé suivant la revendication 7 ou 8,
- dans lequel la mise en fonctionnement (M1) du dispositif (3) de diagnostic in-vitro par le dispositif (5) de commande, comprend une détection (M12) d'un paramètre de diagnostic in-vitro de l'échantillon (S) par le test (M11) de diagnostic in-vitro.

10. Procédé suivant la revendication 9, comprenant en outre :
- une adaptation (M21), par le dispositif (5) de commande, d'un fonctionnement du dispositif (2) d'imagerie médicale sur la base du paramètre de diagnostic in-vitro de l'échantillon (S).

11. Procédé suivant la revendication 9 ou 10, dans lequel l'échantillon (S) est un échantillon de sang, dans lequel le paramètre de diagnostic in-vitro de l'échantillon (S) est un niveau de créatinine de l'échantillon (S), dans lequel le procédé comprend en outre
- calculer (M13), par le dispositif (5) de commande, un taux de filtration glomérulaire estimé sur la base du niveau de créatinine de l'échantillon (S),
- donner (M14), par le dispositif (5) de commande, le taux de filtration glomérulaire estimé.
